# EUROPEAN PATENT APPLICATION

(11) **EP 4 131 276 A1**
(43) Date of publication of application: **08.02.2023**
(21) Application number: 22185980.4
(22) Date of filing: 20.07.2022
(51) Int. Cl.: G16H 10/00

(54) **PATIENT CARE METHODS AND SYSTEMS THROUGH ARTIFICIAL INTELLIGENCE-BASED MONITORING**

(30) Priority: 03.08.2021 KR 20210102100
(71) Applicant: Iti Technology, Jungwon-gu Seongnam-si, Gyeonggi-do 13207 (KR)
(72) Inventor: KIM, Yoon Tae, 13207 SEONGNAM-SI (GYEONGGI-DO) (KR)
(74) Representative: ZBM Patents - Zea, Barlocci & Markvardsen

(57) **Abstract**

A patient care method through artificial intelligence-based monitoring in accordance with one example of the present disclosure comprises steps of: obtaining image information relating a user by a first collecting portion, obtaining speech information relating to the user by a second collecting portion and obtaining biometrics information relating to the user by a third collecting portion (Step 1); representing at least a part of a plurality of information obtained from the first collecting portion, the second collecting portion and the third collecting portion, through a display portion of a user table (Step 2); determining health condition of the user, based on a part of the plurality of information obtained from the first collecting portion, the second collecting portion and the third collecting portion by a server (Step 3); controlling the display portion of the user table to represent a first information automatically generated based on the determined heath condition by the server (Step 4), wherein the first information is converted in real time based on user's feedback on the first information and represents a change in the determined health condition on the display portion.

## Description

### CROSS-REFERENCE TO RELATED APPLICATIONS

This application claims priority and benefit of Korean Patent Application No. 10-2021-0102100 filed on August 03, 2021, in the Korean Intellectual Property Office, the entire disclosure of which is hereby incorporated by reference herein in its entirety.

### BACKGROUND

### Field

The present disclosure relates to patient care methods and systems through artificial intelligence-based monitoring.

### Description of the Related Art

During the challenge of COVID 19 pandemic, new medical systems to which contact-free medical systems are adapted using prior products save expenses by medical staff's effective operations as well as by self-monitoring and self-management through continuous managements according to precision medicine, predictive medicine and preventive medicine, further promoting the national health and achieving business expansions in these fields.

Firstly, as growing interests in aging and health and due to developments in ICT technologies, a paradigm for health care focuses on prevention/customer rather treatment/clinic facility.

In particular, there are increased needs for contact-free/ remote medical services for overcoming medical staff's overwork and risks of COVID-19 infection and ordinary person's emotional instability resulted from COVID-19 pandemic.

Research and development of intelligent conversational agents (chatbot) in charge of initial demand for medical services and industrialization thereof are in progress actively.

Further, big data, Artificial Intelligence, mobile/wearables are technical elements occupying 80% of digital health care.

Further, there are prospects that the average annual increase rate in the remote medical service will reach 14.7% and the global market thereof will grow upto 155.1 billion dollars by the year 2027. It is predicted that there are improvement effects on the health and medical indicators, such as saving of medical expenses and reductions in mortality.

Meanwhile, in societies where shown are increases in aged persons and people who need emotional stability and where regions with the medical vacuum still exist, demanded is a system that communes with and monitors a patient and support their living.

Further, there are continuous increases in morbidity rate (particularly in connection with psychical disorder such as dementia, depression or the like) besides social problems due to elder-neglect resulting from changes in residential status(e.g. nuclear family): lack of social interaction.

Further, notwithstanding adoption of nursing care worker supporting system solving such problems partially, it happens sometimes that residential environment of aged persons becomes even worse because of interaction with others.

Further, emotion-based personalized conversational agents are required, which are human-like and sustainable.

Further, in the modern society where infectious diseases may spread, required are emotional services that make contact-free medical treatment possible and thus allow adoption of remote medical services and AI medical assistants, so as to reduce medical staff's workload and infection risk.

Further, required is a solution to reduce medical staff's workload by accumulating data for individual's usual health/emotional states to provide the data to medical institutions, if necessary and by preliminary verifying the data through emotional services using questionnaires similar to those likely to be questioned by the medical staff when contact-free medical trreatments and remote medical services are necessary.

### [Prior art Documents]

### [Patent Documents]

(Patent Document 1) Korean Patent Application No. 10-2020-0167802
(Patent Document 2) Korean Patent Application No. 10-2020-0110134

### SUMMARY

The present disclosure intends to suggest patient care methods and systems through artificial intelligence-based monitoring in order to solve the aforementioned conventional problems.

Particularly, the present disclosure intends to suggest patient care methods and systems through artificial intelligence-based monitoring which include a first collecting portion for obtaining image information relating a user, a second collecting portion for obtaining speech information relating to the user, a third collecting portion for obtaining biometrics information relating to the user, a user table having a display portion, and a server that determines health condition of the user, based on a part of the plurality of information obtained from the first collecting portion, the second collecting portion and the third collecting portion.

Particularly, according to the present disclosure, a first information automatically generated based on determined health condition may be represented on the display portion of the user table, and the first information changed in real time based on user's feedback and the determined health condition can be represented on the display portion.

According to the present disclosure, an artificial intelligence emotional service can be provided which makes a patient restful to the spirit by applying image information(facial expression, etc.), speech information(speech melody, etc.), context(conversation context through speech recognition, etc.) and bio-signals(electrocardiogram, oxygen saturation, ect).

Further, compared to existing commercialized speech recognition services, as adopting a conversational agent technology provided with human-like emotion and intelligent understanding of the situation to medical services, medical expenses can be saved due to emotional stability resulting therefrom.

Meanwhile, technical solutions to be achieved by the present disclosure are not limited to the aforementioned suggestions, and other not-mentioned technical solutions may be clearly understood by those skilled in the art to which the present disclosure pertains from the description below.

A patient care system through artificial intelligence-based monitoring in accordance with one example to achieve the aforementioned solutions may include a first collecting portion that obtains image information relating a user; a second collecting portion that obtains speech information relating to the user; a third collecting portion that obtains biometrics information relating to the user; a user table has a display portion which represetns at least a part of a plurality of information obtained from the first collecting portion, the second collecting portion and the third collecting portion; and a server that determines health condition of the user, based on a part of the plurality of information obtained from the first collecting portion, the second collecting portion and the third collecting portion, wherein the server may control the display portion of the user table to represent a first information automatically generated based on the determined heath condition, the first information is changed in real time based on user's feedback on the first information and a change in the determined health condition, so as to be represented on the display portion.

Further, the server may determine a first emotion of the user based on the image information, a second emotion of the user based on the speech information and a third emotion of the user based on the biometrics information, and may generate the first information based on at least one of the first emotion, the second emotion and the third emotion.

Further, the server additionally may determine a fourth emotion of the user based on feedback of the user on the first information, and may change the first information based on the fourth emotion.

Further, the server may determine the first emotion based on facial expression of the user, the second emotion based on speech melody of the user and the fourth emotion based on contextual information on the feedback of the user.

Further, the server may transmit information for warning a health risk when matching the determined health condition with any one of a plurality of predetermined health risk conditions, and may controls the information for warning a health risk so as to be represented on the display portion.

Further, the server may generate information for predicting a disease relating to the user based on the image information, the speech information, the biometrics information, information for the first, second, third and fourth emotions, and information for the feedback of the user.

Further, the server may accumulate and store the image information, the speech information, the biometrics information, the information for the first, second, third and fourth emotions, the information for the feedback of the user and the information for predicting a disease relating to the user, and may provide a plurality of the accumulated information to a predetermined medical institution.

Further, the patient care system through artificial intelligence-based monitoring may be used in at least one of contact-free medical treatments and remote medical services.

Further, the first collecting portion may include a camera, a wide angle camera and an infrared camera to take a photograph of the facial expression of the user, and the image information may an image relating to the user at a negative pressure room, an intensive care unit, a general ward and a screening station and home.

Further, the third collecting portion may include an auditory information collecting portion that collects auditory information of the user, a gait information collecting portion that collects gait information of the user, a stress collecting portion that collects stress information of the user, an electrocardiogram(ECG) information collecting portion that collects ECG information of the user, a sleep information collection portion that collects sleep information of the user, a concentration information collecting portion that collects concentration information of the user, an electroencephalogram(EEG) information collection portion that collects EEG information of the user, an oxygen saturation information collecting portion that collects oxygen saturation formation of the user, and a temperature collecting portion that collects temperature information of the user.

Further, the user table may include: a panel portion that has a display pad; a supporting frame that supports the panel portion; and an angle-adjustable connecting portion that connects the panel portion and the supporting frame so as to adjust an angle of the panel portion at the supporting frame with an external force above a set force.

Further, the angle-adjustable connecting portion may include: a guide shaft portion that is fitted into the supporting frame; a rotation bracket that is provided to the panel portion so as to fit the guide shaft portion thereinto; and a binding guide portion that guides the rotation bracket so as to be bound movably along a circumference surface of the guide shaft portion.

Meanwhile, a patient care method through artificial intelligence-based monitoring in accordance with another example to achieve the aforementioned solutions may include steps of: obtaining image information relating a user by a first collecting portion, obtaining speech information relating to the user by a second collecting portion and obtaining biometrics information relating to the user by a third collecting portion (Step 1); representing at least a part of a plurality of information obtained from the first collecting portion, the second collecting portion and the third collecting portion, through a display portion of a user table (Step 2); determining health condition of the user, based on a part of the plurality of information obtained from the first collecting portion, the second collecting portion and the third collecting portion by a server (Step 3); controlling the display portion of the user table to represent a first information automatically generated based on the determined heath condition by the server (Step 4), wherein the first information is converted in real time based on user's feedback on the first information and represents a change in the determined health condition on the display portion.

Further, the step 4 may include steps of: determining a first emotion of the user based on the image information by the server (Step 41); determining a second emotion of the user based on the speech information by the server (Step 42); determining a third emotion of the user based on the biometrics information by the server (Step 43); generating the first information based on at least one of the first emotion, the second emotion and the third emotion by the server (Step 44); additionally determining a fourth emotion of the user based on feedback of the user on the first information by the server (Step 45); and changing the first information based on the fourth emotion by the server (Step 46).

Further, the server may determine the first emotion based on facial expression of the user, the second emotion based on speech melody of the user, and the fourth emotion based on contextual information on the feedback of the user, and, following the step 4, when matching the determined health condition with any one of a plurality of predetermined health risk conditions, the server may further include a step of: transmitting information for warning a health risk and controlling the information for warning a health risk so as to be represented on the display portion (Step 5).

Further, following the step 5, the server may further include a step of generating information for predicting a disease relating to the user based on the image information, the speech information, the biometrics information, information for the first, second, third and fourth emotions, and information for the feedback of the user (Step 6).

### Advantageous Effect

In order to solve the aforementioned existing problems, the present disclosure is capable of providing patient care methods and systems through artificial intelligence-based monitoring.

Particularly, according to the present disclosure, it is capable of providing patient care methods and systems through artificial intelligence-based monitoring which include a first collecting portion for obtaining image information relating a user, a second collecting portion for obtaining speech information relating to the user, a third collecting portion for obtaining biometrics information relating to the user, a user table having a display portion, and a server that determines health condition of the user, based on a part of the plurality of information obtained from the first collecting portion, the second collecting portion and the third collecting portion.

Particularly, according to the present disclosure, it is capable of representing a first information automatically generated based on determined health condition on the display portion of the user table, and of representing the first information changed in real time based on user's feedback and the determined health condition on the display portion.

According to the present disclosure, it is capable of providing an artificial intelligence emotional service which makes a patient restful to the spirit by applying image information(facial expression, etc.), speech information(speech melody, etc.), context(conversation context through speech recognition, etc.) and bio-signals(electrocardiogram, oxygen saturation, ect).

According to the present disclosure, compared to existing commercialized speech recognition services, it is capable of saving medical expenses due to emotional stability resulting therefrom as adopting a conversational agent technology provided with human-like emotion and intelligent understanding of the situation (surrounding circumstances, mind, emotion, tendency, experience, intend, etc.) to medical services.

According to the present invention, it is capable of providing a system that communes with and monitors a patient and support their living in societies where shown are increases in aged persons and people who need emotional stability and where regions with the medical vacuum still exist.

According to the present invention, emotional services make contact-free medical treatment possible in the modern society where infectious diseases may spread, thereby allowing adopting remote medical treatment and AI medical assistants, so as to reduce medical staff's workload and infection risk.

According to the present disclosure, it is capable of accumulating data for individual's usual health/emotional states to provide the data to medical institutions, if necessary and of preliminary verifying the data through emotional services using questionnaires similar to those likely to be questioned by the medical staff when contact-free medical treatment and remote medical services are necessary, thereby reducing medical staff's workload.

According to the present disclosure, it is capable of increasing degree of completion of a conversational agent technology have to 'human-like emotion' and 'intelligent understanding of the situation' which satisfy patients, thereby being adopted to medical services.

Artificial intelligence requires for a technology that understands surrounding circumstances and user's mind (emotion, tendency, experience, intend, etc.) to help human beings. However, person's mind is delicately complex and hardly expressed outwardly. Thus, there are limits in increasing accuracy with noninvasive sensors (e.g. a camera, a microphone, an accelerometer, etc.). According to the present disclosure, it is capable of providing multi-modal learning data and a recognition technology, thereby distinguishing delicate differences.

Currently, in the country, healthcare monitoring is a new wave that is cannot avoidable and thus the study for establishment of a system is ongoing so as to secure competitiveness in the global market through deregulation according to external environment changing so rapidly. In addition, in foreign countries, existing face to face treatments cause the problem of costs. Furthermore, in advanced countries such as the Americas, Australia, etc., this causes big social issues and financial problems. Thus, those countries are in the progress of adopting telehealth to increasing efficiency and reduce medical expenses. Accordingly, it is expected that the present disclosure will have market power.

Meanwhile, advantageous effects to be obtained by the present disclosure are not limited to the aforementioned effects, and other not-mentioned advantageous effects may be clearly understood by those skilled in the art to which the present disclosure pertains from the description below.

### BRIEF DESCRIPTION OF THE DRAWINGS

FIG. 1 shows one example for a block diagram of a patient care system through artificial intelligence-based monitoring, in accordance with the present disclosure.
FIGS. 2A to 2C show specific forms of a user table, in accordance with the present disclosure.
FIGS. 3 to 4 show specific examples for the use of a user table, in accordance with the present disclosure.
FIG. 5 shows one example for the use of a patient care system through artificial intelligence-based monitoring, in accordance with the present disclosure.
FIG. 6 show one example for a monitoring method based on emotion, in accordance with the present disclosure.
FIG. 7 shows one example for the use of a patient care system through artificial intelligence-based monitoring using a plurality of apparatuses, in accordance with the present disclosure.
FIG. 8 shows use of service models according to the present disclosure.
FIG. 9 shows a flowchart explaining a patient care method through artificial intelligence-based monitoring in accordance with the present disclosure.
FIG. 10 shows a flowchart of a method of monitoring based on emotion determination, in accordance with the present disclosure.
FIG. 11 show a flowchart of a method for recognition of a risk situation and predicting user's disease, in accordance with the present disclosure.
FIG. 12 shows a specific example for industrial effects according to the present disclosure.
FIG. 13 shows another example for monitoring bio-signals using a band sensor.
FIG. 14 show a view explaining a scenario which medical staff can take based on monitoring.
FIG. 15 shows one embodiment according to the present disclosure.

### DETAILED DESCRIPTION OF THE PREFERRED EMBODIMENT

Hereinafter, used are terms "module" and "portion" only in the light of easiness for the preparation of a specification, but they neither mean such things distinguishable from each other nor have any act by themselves.

### Patient care system through artificial intelligence-based monitoring

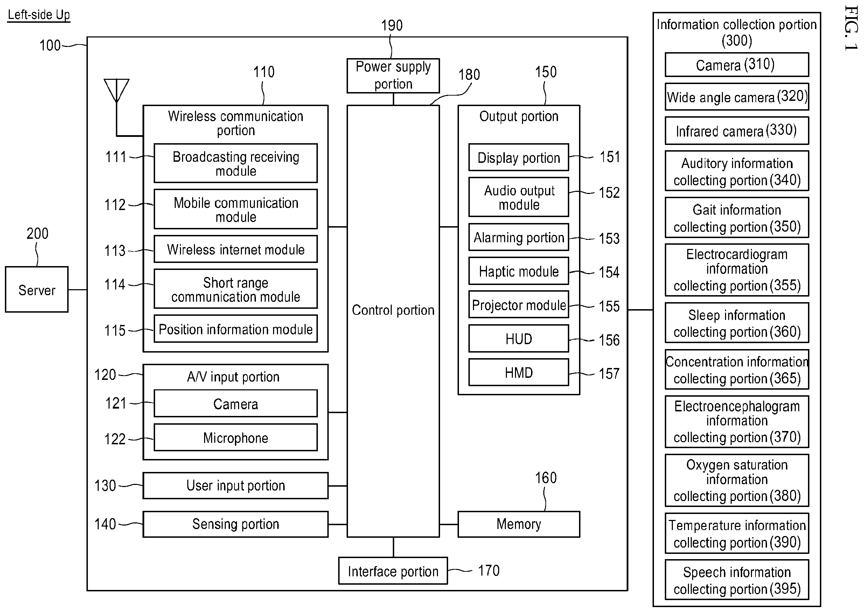

FIG. 1 shows one example for a block diagram of a patient care system through artificial intelligence-based monitoring, in accordance with the present disclosure.

Referring to FIG. 1, a patient care system through artificial intelligence-based monitoring may include a user table 100, a server 200 and an information collecting portion 300.

Firstly, the user table 100 may include a wireless communication portion 110, an Audio/Video(A/V) input portion 120, a user input portion 130, a sensing portion 140, an output portion 150, a memory 160, an interface portion 170, a control portion 180 and a power supply portion 190, etc.

However, configuration elements as shown in FIG. 1 are not essential and thus the patient care system through artificial intelligence-based monitoring may be implemented which has more or less configuration elements.

Hereinafter, the configuration elements above are described in detail one by one.

The wireless communication portion 110 may include at least one module which allows a wireless communication either between the patient care system through artificial intelligence-based monitoring and a wireless communication system or between apparatuses.

For example, the wireless communication portion 110 may include a mobile communication module 112, a wireless internet module 113, a short range communication module 114 and a position information module 115, etc.

A broadcasting receiving module 111 receives a broadcasting signal and/or broadcasting related information from an external broadcasting management server through broadcasting channels.

The broadcasting channel may include a satellite channel and an on-air channel. The broadcasting management server may signify a server that generates and transmits a broadcasting signal and/or broadcasting related information, alternatively signifying a server that receives a pre-generated broadcasting signal and/or broadcasting related information and transmit this to the user table 100. The broadcasting signal may include a TV broadcasting signal, a radio broadcasting signal, a data broadcasting signal, as well as a broadcasting signal in a combination form of the preceding.

The broadcasting related information may signify information relating to a broadcasting channel, a broadcasting program or a broadcasting service provider. The broadcasting related information may be also provided through a mobile communication network and, in such a case, may be received by the mobile communication module 112.

The broadcasting related information may exist in various forms, for example, electronic program guide(EPG) of digital multimedia broadcasting or electronic service guide(EGS) of digital video broadcast-handled(DVB-H), etc.

The broadcasting receiving module 111 may receive a digital broadcasting signal by using a digital broadcasting system, such as digital multimedia broadcasting-terrestrial(DMB-T), digital multimedia broadcasting-satellite(DMB-S), media forward link only(MediaFLO), digital video broadcast-handheld(DVB-H), DVB-CBMS, OMA-BCAST, integrated services digital broadcast-terrestrial(ISDB-T), etc. Further, the broadcasting receiving module 111 may be configured to be suitable for the aforementioned digital broadcasting system as well as other broadcasting systems.

The broadcasting signal and/or broadcasting related information that were received through the broadcasting receiving module 111 may be stored in the memory 160.

The mobile communication module 112 transmits and receives a wireless signal with at least one of a base station, the external user table 100 and a server on a mobile communication network. The wireless signal may include various forms of data according to transmitting and receiving of a speech call signal, a vide call signal or a text/multimedia message.

The wireless internet module 113 refers to a module for a wireless internet connection, and may be built in or on the exterior of the user table 100.

Wireless Wireless LAN(WLAN)(Wi-Fi), wireless broadband(Wibro), world interoperability for microwave access(Wimax), high speed downlink packet access(HSDPA), etc. may be used as a technology for the aforementioned wireless internet.

The short range communication module 114 refers to a module for short range communication. Bluetooth, radio frequency identification(RFID), infrared data association(lrDA), ultra-wideband(UWB), ZigBee, etc. may be used as a technology for the short range communication.

The position information module 115 is a module for obtaining a position of the user table 100 and a representative example thereof is a global position system(GPS) module. According to the modern technology, the GPS module 115 calculates distance information away from at least three satellites and an accurate time information and then applies trigonometry to the calculated information, allowing accurate calculation of three dimensional position information depending on latitude, longitude and altitude. Currently, widely used is a method to calculate position and time information using three satellites and to calibrate an error on the calculated position and time information using one more satellite. Further, the GPS module 115 continuously calculates a current position in real time to calculate velocity information.

Referring to FIG. 1, the A/V input portion 120 is for inputting an audio signal or a video signal and may include a camera 121, a microphone 122, etc.

The camera 121 processes an image frame such as a still image, a movie or etc. that was obtained by an image sensor on the photographing mode and may represent a processed image frame on a display portion 151.

The processed image frame in the camera 121 may be stored in a memory 160 or transmitted to the outside through the wireless communication portion 110.

At least two cameras 121 may be provided according to a user environment.

The microphone 122 receives an input external audio signal by a microphone on recording mode, speech recognition mode, etc. and processes this signal to electrical speech data.

The processed speech data is converted into a form that is transmittable to a mobile communication base station through the mobile communication module 112, and then may be output.

Various noise removal algorithms for removing noises that occur during the receipt of the input external audio signal may be implemented in the microphone 122.

Next, the user input portion 130 generates input data for a user to control operation of the patient care system through artificial intelligence-based monitoring. The user input portion 130 may be composed of a key pad, a dome switch, a touch pad(static pressure/static electricity), a jog wheel, etc.

The sensing portion 140 generates a sensing signal for controlling the operation of the patient care system through artificial intelligence-based monitoring by sensing a current state of the patient care system through artificial intelligence-based monitoring, such as a switching state of the patient care system through artificial intelligence-based monitoring, user's touch or not, a bearing of the patient care system through artificial intelligence-based monitoring, acceleration/ deceleration of the patient care system through artificial intelligence-based monitoring, etc.

The sensing portion 140 may sense power supply of the power supply portion 190 or not, connection of the interface portion 170 to an external apparatus or not, etc.

Meanwhile, the output portion 150 is to generate an output relating to a sense of sight, hearing, touch or etc. and thus may include the display portion 151, an audio output module 152, an alarming portion 153, a haptic module 154 and a projector module 155, a head-up display(HUD), a head mounted display(HMD), etc.

The display portion 151 represents(outputs) information that was processed in the patient care system through artificial intelligence-based monitoring.

The display portion 151 may include at least one of a liquid crystal display(LCD), a thin film transistor-liquid crystal display(TFT LCD), an organic light-emitting diode(OLED), a flexible display, a 3D display, etc.

A part of these displays may be formed into a transparent type or a light transmissive type, thus allowing seeing the outside therethrough. This refers to a transparent display and a representative example thereof is a transparent OLED(TOLED), etc. A rear structure of the display portion 151 may be also formed into a light transmissive type structure. Due to this structure, a user may see an object positioned on the rear of the patient care system through artificial intelligence-based monitoring, through an area occupied by the display portion 151 in the body of the patient care system through artificial intelligence-based monitoring.

At least two display portions 151 may exist, depending on an implement of the patient care system through artificial intelligence-based monitoring. For example, a plurality of display portions may be positioned apart from each other or in a line on one side in the patient care system through artificial intelligence-based monitoring. Further, these may be positioned on different sides, respectively.

When the display portion 151 and a sensor that senses a touch action (hereinafter, referred to as a 'touch sensor') mutually form a layer structure (hereinafter, referred to as a 'touch screen'), the display portion 151 may be used as an input device besides an output device. The touch sensor may have a form, for example, a touch film, a touch sheet, a touch pad, etc.

The touch sensor may be configured to convert a change in a pressure applied to a specific region of the display portion 151 or static electricity occurring in a specific region of the display portion 151, etc. into an electrical input signal. The touch sensor may sense touched position and area as well as an input when touched.

When a touch input for the touch sensor occurs, signal(s) corresponding thereto is sent to a touch controller. The touch controller processes those signal(s) and then transmits relevant data to the control portion 180. The control portion 180 hereby sees what area of the display portion 151 was touched is.

A proximity sensor 141 may be positioned in an internal area of the patient care system through monitoring that was enclosed with the touch screen, or in the vicinity of the touch screen. The proximity sensor refers to a sensor that detects existence or nonexistence of an object approaching a predetermined detecting side or an object in the vicinity by using an electromagnetic force or an infrared ray without any mechanical contact. The lifespan of the proximity sensor is longer than a contact typed sensor and the utilization thereof is also high.

Examples of the proximity sensor include a transmission type of photoelectric sensor, a direct reflection type of photoelectric sensor, a mirror reflection type of photoelectric sensor, a high frequency oscillation type of proximity sensor, a capacitive proximity sensor, a magnetic proximity sensor, an infrared proximity sensor, etc. When the touch screen is an electrostatic type, this is configured to detect proximity of a pointer by a change in an electric field depending on the pointer's approaching. In this case, the touch screen(touch sensor) may be classified as a proximity sensor.

Hereinafter, for the convenience of description, a behavior that recognizes a pointer in the proximity of the touch screen without contacting this as being positioned on the touch screen is referred to as "proximity touch". Further, a behavior in which the pointer actually contacts the touch screen refers to "contact touch". A position where a proximity touch of the pointer is allowable on the touch screen signifies such a position where the pointer vertically corresponds to the touch screen when proximately touched.

The proximity sensor senses a proximity touch and a proximity touch pattern(for example, a proximity touch distance, a proximity touch direction, a proximity touch speed, a proximity touch time, a proximity touch position, a proximity touch movement, etc.). Information corresponding to the sensed proximity touch action and proximity touch pattern may be output on the touch screen.

The audio output module 152 may output audio data that was received from the wireless communication portion 110 on recording mode, speech recognition mode, broadcasting receiving mode, etc. or stored in the memory 160.

The audio output module 152 outputs an audio signal relating to a function performed in the patient care system through monitoring. This audio output module 152 may include a receiver, a speaker, a buzzer, etc.

The alarming portion 153 may output a signal for notifying occurrence of events in the patient care system through monitoring.

The alarming portion 153 may output a signal for notifying occurrence of those events in a different form, for example, a vibration, besides a video signal or an audio signal.

The video signal or audio signal may be output through the display portion 151 or the speech output module 152. Thus, these 151, 152 may be classified as a part of the alarming portion 153.

The haptic module 154 generates various tactile effects which a user can feel. A representative example of tactile effects generated from the haptic module 154 is a vibration. An intensity, a pattern, etc. of the vibration generated from the haptic module 154 may be controlled.

For example, different vibrations may be synthesized and output, or may be output successively.

Besides a vibration, the haptic module 154 may generate various tactile effects including an effect resulting from stimulation such as spray injection power or suction power through a pin arrangement that vertically moves with respect to a contacted skin surface, an spray injection port or a suction port, brush on a skin surface, contact of an electrode, electrostatic force, etc., an effect resulting from recreation of cold/warm feelings using elements capable of absorbing or generating heat, etc.

The haptic module 154 may be implemented to transmit a tactile effect through direct contact as well as muscular senses of user's fingers and arms. At least two haptic modules 154 may be provided according to an aspect of the present disclosure.

The projector module 155 is a configuration element for performing an image project function and may display, on an external screen or wall, an image which is the same as or at least part of which an image represented on the display portion 151 according to the control portion 180, or at least a part of which is different therefrom.

Particularly, the projector module 155 may include a light source (not illustrated) that generates light for outputting an image to the outside (for example, a laser light beam), an image generating unit (not illustrated) that generates an image to be output to the outside using the light generated from the light source, and a lens (not illustrated) that expands and outputs the image at a predetermined focal distance to the outside. Further, the projector module 155 may include a device (not illustrated) capable of adjusting an image projection direction by mechanically moving the lens or the entire module.

The projector module 155 may be classified into a cathode ray tube(CRT) module, a liquid crystal display(LCD) module, a digital light processing(DLP) module, etc. according to the type of an element of the display unit. Especially, the DLP module may be advantageous in miniaturization of the projector module 151 in a way to expand and project the image generated by reflecting the light generated from the light source on a digital micromirror device(DMD) chip .

Preferably, the projector module 155 may be provided at a side, a front side or a rear side of the patient care system through monitoring in a longitudinal direction. Definitely, the projector module 155 may be also provided at any position of the patient care system through monitoring, if necessary.

Further, a head-up display(HUD) 156 signifies a device that projects information for a current vehicle speed, a residual fuel amount, a navigation guide, etc. into a graphic image on the window in front of a driver in a vehicle.

Further, a head mounted display(HMS) 157 is a representative device that outputs virtual reality information.

Virtual reality is a generic term for an interface between a human and a computer, which prepares a 3D content for a certain environment or circumstance to make a user of the 3D content feel like interacting with a real surrounding circumstances or environment.

Generally, a three-dimensional effect perceived by a person results from combination of a degree of a change in the thickness of a crystalline lens, a difference in an angle between both eyes and an object, differences in the positon and shape of the object shown in right and left eyes, a time difference according to the movement of the object, effects of various mentalities and memories, etc.

The most key factor making a person feel a three dimensional effect is a binocular disparity appearing when both eyes of the person apart about 6.5cm in a transverse direction. That is, the person looks at an object with an angle difference caused by a time difference between both eyes, this difference resulting in different images come into the respective eyes. Two images are transmitted to a brain through a retina, the brain amalgamating information for the two images accurately to allow the person to feel a 3D image.

Such a 3D content has been widely used in the field of various media already and received a favorable review from consumers. For example, a 3D movie, a 3D game and an experience-display are representative.

It is diversely demanded to popularize a 3D content according to the virtual reality technology and to develop a technology for providing virtual reality services accompanied with higher-level immersion of a user.

Generally, an image display device refers to an image-representing device that forms a focus to form a virtual big-screen at a far distance with an image light generated at a very proximate position to eyes by using a precise optical device, thus allowing a user to see an enlarged virtual image.

Further, the image display device may be sorted into a see-close type allowing a user to see not a surrounding environment but an image light emitted from a display element only, and a see-through type allowing the user to see the surrounding environment through a window and the image light emitted from the display element at the same time.

The HMD 157 refers to any of various digital devices which allows a user to receive a multimedia content by wearing on the head like glasses. According to current trends to lighten the weight of and to miniaturize a digital device, various wearable computers are developed and HMDs widely used are.

For example, when a microphone and a speaker are mounted on the HMD 157, a user wearing the HMD 157 may have a phone call. Further, for example, when an infrared camera 122 is mounted on the HMD 157, the user wearing the HMD 157 may capture a desired direction of image.

Further, the memory 160 may store a program for processing and controlling the control portion 180 and perform a function for temporarily storing input/output data (for example, a text message, an audio, a still image, a movie, etc.). The memory 160 may also store a usage frequency for the respective data. Further, the memory 160 may store data relating to various patterns of a vibration and an audio that were output when performing a touch input on the touch screen.

The memory 160 may include at least one storing medium selected from a flash memory, a hard disk type of memory, a multimedia card micro type of memory, a card type of memory(for example, SD or XD memory, etc.), a Random Access Memory(RAM), a Static Random Access Memory(SRAM), a Read-Only Memory(ROM), an Electrically Erasable Programmable Read-Only Memory(EEPROM), a Programmable Read-Only Memory(PROM), a magnetic memory, a magnetic disc and an optical disc. The patient care system through monitoring may be operated in association with a web storage that performs a storing function of the memory 160 on the internet.

The interface portion 170 serves as a connecting passage to all external apparatuses that are connected to the patient care system through monitoring. The interface 170 receives data or power from an external apparatus to transmit the received data or power to respective configuration elements inside the patient care system through monitoring or to transmit data inside the patient care system through monitoring to the external apparatus. For example, the interface portion 170 may include a wire/wireless headset port, an external charger port, a wire/wireless data port, a memory card port, a port connecting a device having an identification module, an audio input/output(I/O) port, a video input/output(I/O) port, an earphone port, etc.

The identification module is a chip that stores various kinds of information for authenticating the use of the patient care system through monitoring, and may include a user identify module(UIM), a subscriber identify module(SIM), a universal subscriber identity module(USIM), etc. A device having the identification module (hereinafter, referred to as a 'identification device') may be manufactured into a smart card type. Thus, the identification device may be connected with the patient care system through monitoring through a port.

The interface portion may be a passage for supplying power from external cradles to the patient care system through monitoring when the patient care system through monitoring is connected to the cradles, or a passage for transmitting various command signals that were input from the cradles by a user to the mobile apparatus. The various command signals or power that were input from the cradles may serve as a signal for recognizing whether the mobile apparatus is accurately mounted on the cradles or not.

The control portion 180 generally controls overall operation of the patient care system through monitoring.

The power supply portion 190 is applied with external power and internal power by controlling by the control portion 180 to supply power required for operating respective configuration elements.

Various embodiments described herein may be implemented in a recoding medium that is readable by a computer or a device similar thereto, for example, by using a software, a hardware or a combination of the preceding.

According to an implementation in aspect of hardware, an embodiment described herein may be implemented by using at least one of application specific integrated circuits(ASICs), digital signal processors(DSPs), digital signal processing devices(DSPDs), programmable logic devices(PLDs), field programmable gate arrays(FPGAs), processors, controllers, micro-controllers, microprocessors and electrical units for performing other functions. In some cases, embodiments described herein may be implemented as the control portion 180 itself.

According to an implementation in aspect of software, embodiments, such as procedures and functions, described herein may be implemented as separate software modules. The respective software modules may perform one or more functions and operations described herein. A software code may be implemented with a software application written in an appropriate programming language. The software code is stored in the memory 160 and may be performed by the control portion 180.

Further, the server 200 builds database and may exchange information with the user table 100.

At this time, a short range communication or a long range communication may be applied between the server 200 and the user table 100.

Further, a wireless communication technology usable herein may include wireless LAN(WLAN)(Wi-Fi), wireless broadband(Wibro), world interoperability for microwave access(Wimax), high speed downlink packet access(HSDPA), etc.

Further, a shot range communication technology may include bluetooth, radio frequency identification(RFID), infrared data association(lrDA), ultra-wideband(UWB), ZigBee, etc.

Further, the information collecting portion 300 may include a camera 310, a wide angle camera 320 and an infrared camera 330, an auditory information collecting portion 340, a gait information collecting portion, an electrocardiogram information collecting portion 355, a sleep information collection portion 360, a concentration information collecting portion 365, an electroencephalogram information collection portion 370, an oxygen saturation information collecting portion 380, a temperature collecting portion 390, a speech information collecting portion 395, etc.

The information collecting portion 300 is widely categorized as a first collecting portion for obtaining user related image information, a second collecting portion for obtaining user related speech information and a third collecting portion for obtaining biometrics information.

The first collecting portion may include the camera 310, the wide angle camera 320 and the infrared camera 330.

Further, the second collecting portion may include the speech information collecting portion 395.

Further, the third collecting portion may include the auditory information collecting portion 340, the gait information collecting portion 350, the electrocardiogram information collecting portion 355, the sleep information collection portion 360, the concentration information collecting portion 365, the electroencephalogram information collection portion 370, the oxygen saturation information collecting portion 380 and the temperature collecting portion 390.

Further, the information collection portion 300 is applied with a wire communication, a short range communication or a long range communication to exchange information with the server 200 and the user table 100.

At least a part of information that was obtained from the first collecting portion, the second collecting portion and the third collecting portion may be represented on the user table 100.

Further, the serve 200 may determine a health condition of a user based on at least a part of a plurality of information that was obtained from the first collecting portion, the second collecting portion and the third collecting portion.

Especially, the server 200 may control the display portion of the user table to represent a first information that was automatically generated based on the determined heath condition.

The first information is changed in real time based on user's feedback on the first information and a change in the determined health condition, so as to be represented on the display portion.

### User table

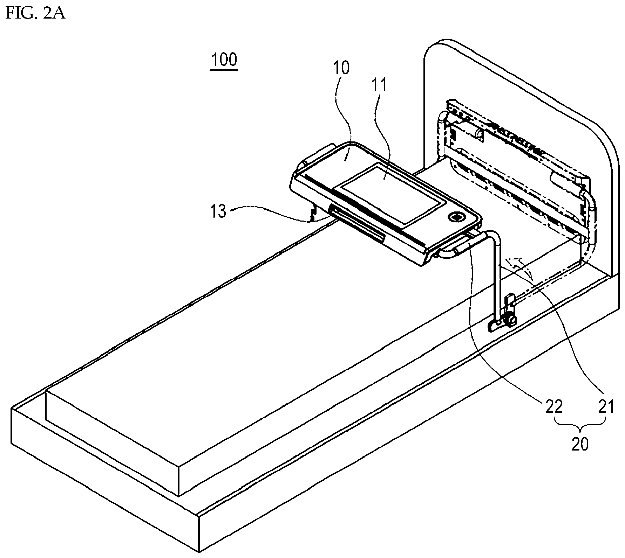

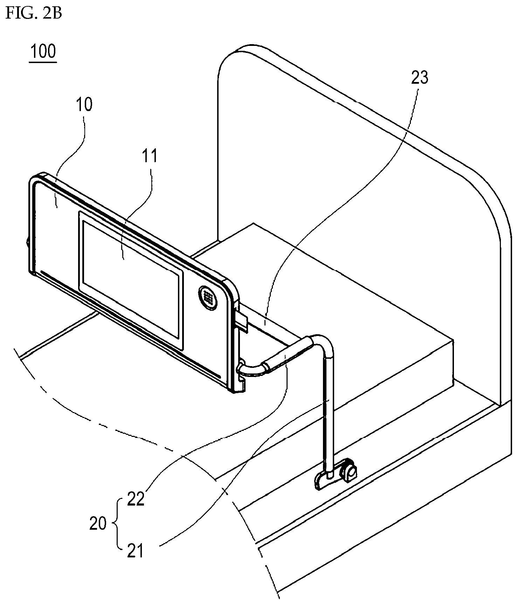

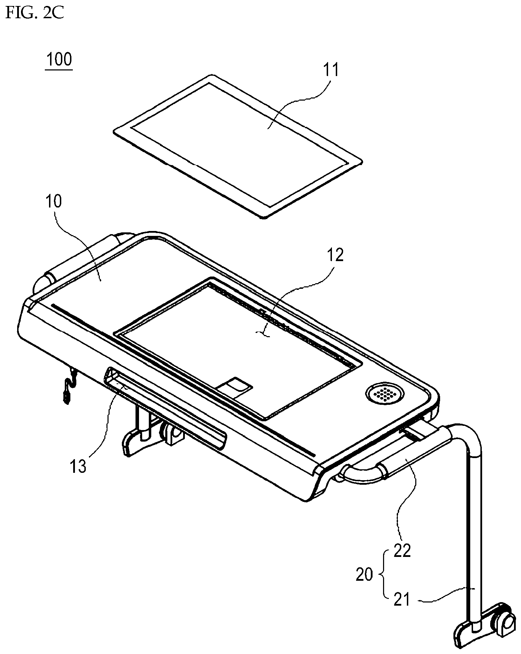

FIGS. 2A to 2C show specific forms of a user table, in accordance with the present disclosure.

Referring to FIGS. 2A to 2C, the user table 100 includes a panel portion 10, a supporting frame 20 and an angle-adjustable connecting portion.

The panel portion 10 has a display pad 11. The display panel 11 is connected with a main server 200 of a medical institute through a network and provides various broadcasting images, advertising contents, applications, etc. provided by the main server 200 to a user. The application may include an application provided from the main server 200 as well as an application provided from various kinds of external organizations, etc.

The main server 200 is installed in a medical institute and manages integrated service operation for various services provided through the medical institute. For this, the main server 200 stores an application for providing services besides patient's medical records, and further provides broadcasting images, etc. to the display panel 11.

Accordingly, the main server 200 provides the detailed information or broadcasting images for an advertising content to the display panel 11 when receiving a request therefor from the display pad 11. Further, once an application is executed, the main server 200 provides various services provided through the application to the display pad 11 for the medical institute.

The advertising content may include such an advertising content provided from the main server 200 or the outside. The application may include various applications provided through a conventional smart terminal besides one for medical use.

Meanwhile, the display pad 11 provides various advertising contents and broadcasting images, a webpage, etc. that are received from the main server 200 to the user, thus allowing the user to use various services.

This display pad 11 may include a communication portion, an advertising processing module 12, a broadcasting image processing module 13, an application processing module, an image processing portion, a touch screen portion, a storing portion, a key input portion and a control portion.

The communication portion is connected with the main server 200 through a network and provides various communication interfaces with the main server 200.

The advertising processing module 12 outputs an advertising content that was input from the communication portion. The advertising content may be provided in various types, such as an image, a movie, an animation, or etc.

The broadcasting processing module 13 processes various kinds of the broadcasting images that are provided through cables and then outputs the processed images. The broadcasting image may include broadcasting images that are cablecasted by various cable TV channels as well as broadcasting images that are provided from broadcasting companies.

The application processing module represents an icon of a pre-installed application and executes the application with response to a control signal of the control portion, thus allowing the user to use various services provided the application.

The image processing portion displays a broadcasting image or an advertising content that was output from the broadcasting image processing module 13, the advertising processing module 12 and the application processing module or the icon through the touch screen portion with dividing a screen.

That is, the image processing portion displays a broadcasting image and advertising content, an icon, etc. on a screen of the touch screen portion on a picture in picture(PIP) mode. In this case, once a broadcasting image, an advertising content, an icon, etc. are selected (as touching or dragging), the image processing portion may represent either the broadcasting image or the advertising content, or an application execution screen on the whole area of the screen or a part thereof.

Additionally, once a relevant screen is selected and dragged while executing either a broadcasting image or advertising content or an application execution screen, the image processing portion may reduce or expand the screen with response thereto, and may further move this to an output area.

The touch screen displays either a broadcasting image or advertising content or an icon on the screen, and also displays either the broadcasting image or advertising content or an application execution screen on the whole screen or a part thereof.

In addition, the touch screen receives user's touch or a drag command that was input therein and then inputs this in the control portion.

The storing portion stores various kinds of information provided during the execution of either a broadcasting image or advertising content or an application that was input from at least one of the broadcasting image processing portion, advertising processing module and the application processing module.

The key input portion is installed in one side of the touch screen and inputs various commands for executing a broadcasting image, an advertising content, an icon, etc. This key input portion may be provided in a type of touch pad. The command input through the key input portion may include a command for executing a broadcasting image, an advertising content and an icon, as well as a command for setting a menu for those broadcasting image, advertising content and icon.

The control portion controls either a broadcasting image or advertising content or an icon that was output from at least one of the aforementioned broadcasting image processing portion, advertising processing module and application processing module so as to be represented through the image processing portion with dividing the screen. Further, once any one of either the broadcasting image or the advertising content, the icon, etc. is touched while provided through the touch screen, the control portion reduce or expands a screen of either a relevant broadcasting image or a relevant advertising content or executes a relevant icon.

In detail, the control portion displays either a broadcasting image or advertising content, or an icon that was output from the aforementioned broadcasting image processing portion, advertising processing module and application processing module on one screen through the touch screen portion.

Once a broadcasting image is touched through the touch screen, the control portion may reduce or expand the size of a relevant broadcasting image, and may further provide menus for changing a channel, adjusting a volume, etc., thus allowing a user to select a relevant menu. Further, such a menu for either changing a channel or adjusting a volume may be performed while providing the broadcasting image on the entire screen as described above as well as on a PIP mode.

Once an advertising content is touched through the touch screen portion, the control portion may reduce or expand a size of a relevant advertising content, and may further provide detailed information for the relevant advertising content. As one example, in a case of the advertising content is an advertisement, a user recognizes a relevant advertisement in more detail by providing detailed information for the relevant advertising content.

Meanwhile, once an icon is touched through the touch screen, the control portion executes an application corresponding to a relevant icon. In this case, the control portion may output an application execution screen on overall area of the touch screen or a part thereof through the image processing portion.

In addition, the control portion executes various menus according to a control command input through the touch screen portion or a control command input through the key input portion, and may set up an arrangement of a broadcasting image, an advertising content and an icon or not, and execution of a screen size thereof or not, through those menus.

Further, the control portion outputs or executes various broadcasting images, advertising content or etc. that was pre-stored in the storing portion according to a control command input from the touch screen portion or the key input portion.

Meanwhile, the control portion provides a webpage to a part of the whole screen of the touch screen through the touch screen portion. Such a webpage is preset and once a webpage is selected through the touch screen, a relevant webpage is provided to the whole screen. At this time, a user select the relevant page and thus is available to use services such as the use or search of information. This webpage may include various webpages besides one provided to a medical institute.

Like this, the control portion controls either a broadcasting image or advertising content and an icon output from at least one of the broadcasting image processing portion 13, the advertising processing module 12 and the application processing module so as to be represent through the touch screen with dividing the screen through the image processing portion. At this time, once a command is input through the touch screen portion or the key input portion, the control portion controls the respective broadcasting image processing module 13, the advertising processing module 12 and the application processing module according to a relevant command. A user, hereby, may use various services provided through the broadcasting image processing module 13, the advertising processing module 12 and the application processing module.

The embodiment as described above is installed in medical institutes such as a hospital, etc. and then outputs various kinds of medical information, a cable image, an advertising content, an application, etc. according to a user's command.

Further, the present embodiment represents applications for using medical information and other services on a screen, allowing a user to select any one of those applications and use various services provided from the selected application.

In addition, the present embodiment provides a logo of a hospital, an advertisement, a broadcasting, etc. in a form of PIP on one screen, allowing the user to be provided with various kinds of broadcasting images, information, or etc. easily.

The panel portion 10 forms a depressed embedding portion so as to embed the display pad 11 therein to be exposed. Further, the panel portion 10 may have a gripping hole for forming a handle.

This display pad 11 may be detachably fixed to the panel portion 10 in various manners, such as hook connection, etc. to prevent arbitrary breakaway from the embedding portion.

The supporting frame 20 supports the panel portion 10. Particularly, the supporting frame 20 performs a role for supporting the panel portion 10 so as that the panel portion 10 is rotated angle-adjustably by the angle-adjustable connecting portion.

The supporting frame 20 includes a longitudinal bar 21 and a transverse bar 22.

A pair of supporting frames 21 parallel with each other is formed and each of the supporting frames is hinge-connected to respective edges in both sides of a corresponding bed frame.

The transverse bar 22 is connected to the longitudinal bar 21, and to the panel portion 10 through the angle-adjustable connecting portion. At this time, a supporting bar may be provided in between the transverse bars 22 to support the panel portion 10 that is laid down.

Meanwhile, the angle-adjustable portion connects the panel portion 10 and the supporting frame 20 so as that the panel portion 10 is angle-adjustable with an external force above a set force in the supporting frame 20.

This angle-adjustable connecting portion includes a guide shaft connected to the supporting frame 20 to be fitted thereinto, a rotation bracket provided to the panel portion 10 to support the guide shat, and a binding guide portion to guide the rotation bracket to be bound movably along a circumference surface of the guide shaft portion.

Meanwhile, FIGS. 2A to 2C show the user table 100 for a bed hospital bed, however, this may be implemented into any of mobile types

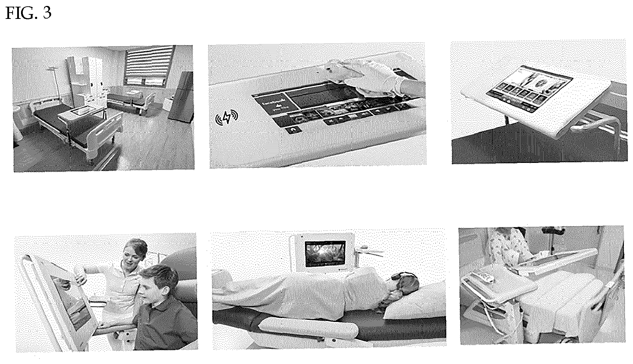

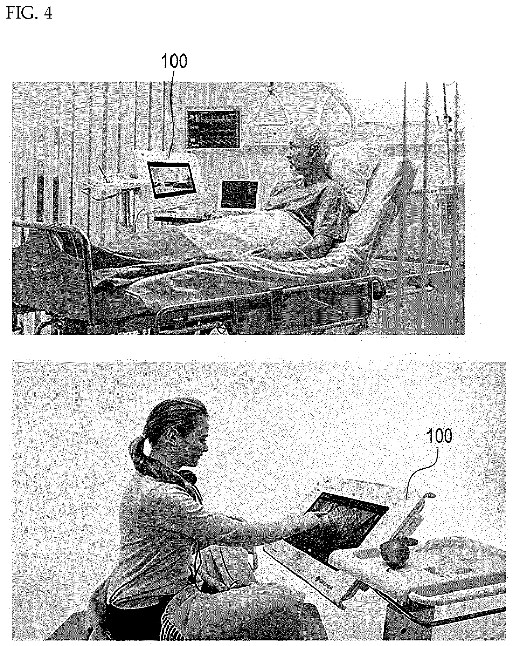

FIGS. 3 and 4 show specific examples for the use of a user table, in accordance with the present disclosure.

That is, the user table is available to be used in any of types such as a fixed type for a multi-person room, an anti-bacterial cleansing and anti-fingerprint type, a bed table-fixed type, a clinic use type, a mobile type, an over-bed type for a single or a double room, etc.

### Utilization of patient care system through artificial intelligence-based monitoring

A patient care system through image/speech/bio-signal and artificial intelligence-based monitoring for remote medical and individual customized services provided in accordance with the present disclosure is an intelligent medical service system (contact-free remote medical service/digital emotional service). This enhances medical staff's contact-free video tele-medical service and a digital emotional service (personalized conversation and care for improving a recognition ability such as HR, emotions, etc.) and provides following three services on behalf of the medical staff.
(1) Patient health care management service
(2) AI medical service
   - Patient condition monitoring
   - Individual patient DB construction
   - Medical video consultancy/remote medical support
(3) AI emotional service
   - Video conversation-based emotional stability
   - Recognition/ memory monitoring and management
   - Digital therapeutics

"(1) Patient health care management service" is an effective life medical management service such as patient-customized services during hospitalization including entertainment, disease education, patient's schedule information, prescription, treatment progression, etc. and nursing information-input and management services.

"(2) AI medical service" monitors patient's condition in 24 hour-day and builds individual database followed by analysis and prediction of prognosis of a disease.

Further, this may be a way for supporting a remote medical service to provide medical consultancy to patient's avatar on a video on behalf of the medical staff within a legally acceptable range.

"(3) AI emotional service" may help a patient to ameliorate emotional distress or depression and thus to return to a healthy life.

That is, an intelligent patient management system understands patient's emotion through natural language processing of facial expression, speech melody and speech recognition of a patient and may keep appropriate conversation to stabilize the patient's emotion considering individual personality.

Further, a change in a recognition ability and memory is monitored by monitoring a patient continuously and may manage the patient by playing a recognition game through conversation, if necessary. This may be one example for digital therapeutics of which the study and practice has been currently started.

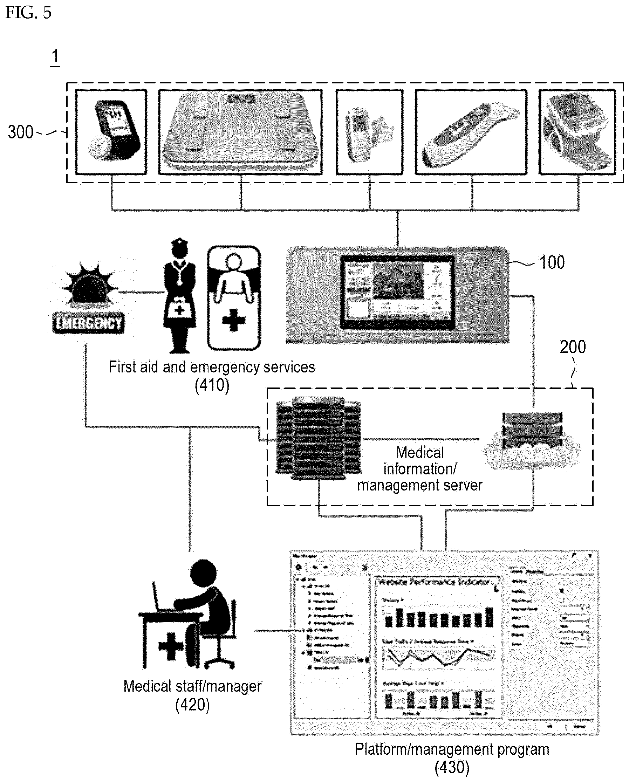

FIG. 5 shows one example for the use of a patient care system through artificial intelligence-based monitoring, in accordance with the present disclosure.

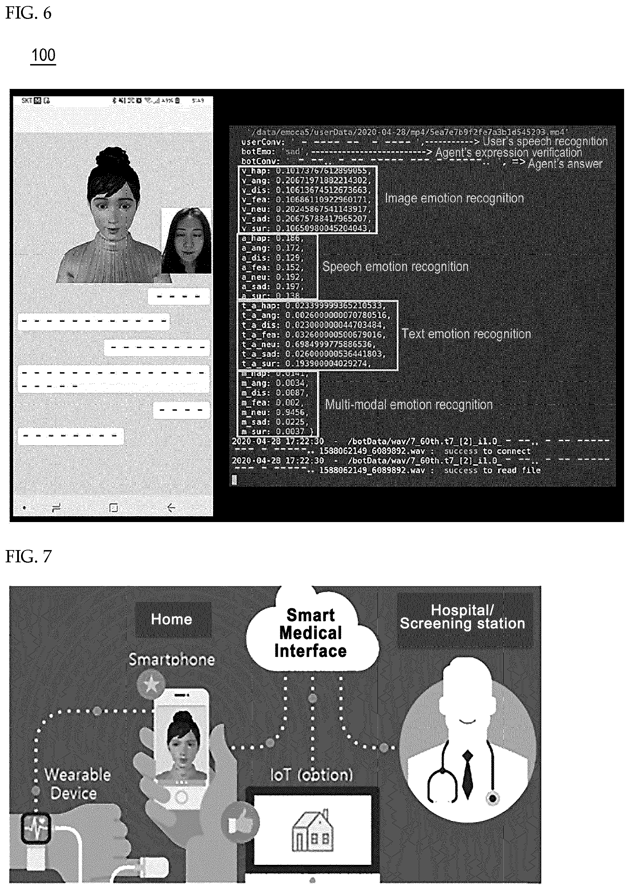

Further, FIG. 6 show one example for a monitoring method based on emotion, in accordance with the present disclosure.

Referring to FIG. 5, a first collecting portion of a patient care system through artificial intelligence-based monitoring may obtain image information relating to a user, a second collecting portion thereof may obtain speech information relating to the user, and a third collecting portion may obtain biometrics information relating to the user.

Further, the display portion 151 of the user table 100 may represent at least a part of a plurality of information obtained from the first collecting portion, the second collecting portion and the third collecting portion.

Further, the server 200 may determine health condition of the user, based on a part of the plurality of information obtained from the first collecting portion, the second collecting portion and the third collecting portion.

Particularly, the server 200 may control a first information automatically generated based on determined health condition so as to be represented on the display portion 151 of the user table.

At this time, the first information is changed in real time based on user's feedback and the determined health condition and may be represented on the display portion 151.

Further, referring to FIG. 6, the server 200 according to the present disclosure may determine a first emotion of a user based on the image information, a second emotion of the user based on the speech information and a third emotion of the user based on the biometrics information.

The server 200 may generate the first information based on at least one of the first emotion, the second emotion and the third emotion.

Particularly, the server 200 may additionally determine a fourth emotion of the user based on feedback of the user on the first information.

The first information may be changed based on the fourth emotion.

The server 200 may determine the first emotion based on facial expression of the user, the second emotion based on speech melody of the user and the fourth emotion based on contextual information on the user's feedback.

Further, the server 200 transmits information for warning a health risk when matching the determined health condition with any one of a plurality of predetermined health risk conditions, and may control the information for warning a health risk so as to be represented on the display portion 151.

Further, the server 200 may generate information for predicting a disease relating to the user based on the image information, the speech information, the biometrics information, information for the first, second, third and fourth emotions, and information for the user's feedback.

Furthermore, the server 200 accumulates and stores the image information, the speech information, the biometrics information, the information for the first, second, third and fourth emotions, the information for the user's feedback and the information for predicting a disease relating to the user and may provide a plurality of the accumulated information to predetermined medical institutions (410, 420, 430).

The patient care system through artificial intelligence-based monitoring, hereby, may be used in at least one of contact-free medical treatment and remote medical service.

As mentioned above, the first collecting portion may include a camera, a wide angle camera and an infrared camera to take a photograph of the facial expression of the user.

When the up-down and left-right operation of the infrared and wide angle cameras is available, it is allowable to place a front-facing camera (operated along with the infrared camera) replacing the wide angle camera at the same.

Further, it is preferred to apply a wide angle of 180 degrees. However, when replacing wide angle camera with the front-facing camera, it is allowable to apply the same viewing angle as that of infrared camera.

The more microphones are applied to the present disclosure, the more an accuracy is increased.

Further, it is available to design the infrared camera and the wide angle camera according to the present disclosure to allow up-down and left-right operations. Typically, an up-down operation is allowable within 100 degrees and so is a left-right operation within 180 degrees.

Further, it is preferred to apply an image angle of the infrared and wide angle cameras being 120 degrees.

Further, a viewing angle of the infrared camera is applied being D=90° H=80° V=60° and a viewing angel of the wide angle camera is applicable being D=180°.

At this time, the image information may be an image relating to the user at a negative pressure room, an intensive care unit, a general ward and a screening station and home.

Further, the third collecting portion may include an auditory information collecting portion that collects auditory information of the user, a gait information collecting portion that collects gait information of the user, a stress collecting portion that collects stress information of the user, an electrocardiogram(ECG) information collecting portion that collects ECG information of the user, a sleep information collection portion that collects sleep information of the user, a concentration information collecting portion that collects concentration information of the user, an electroencephalogram(EEG) information collection portion that collects EEG information of the user, an oxygen saturation information collecting portion that collects oxygen saturation formation of the user, a temperature collecting portion that collects temperature information of the user and a speech information collecting portion that collects speech information of the user.

As described above, key technologies of the present disclosure are as follows.
(1) General personal smart pad having display and communication functions
(2) Three kinds of cameras (for facial expression recognition, surrounding circumstance recognition, infrared bio-signal recognition)
(3) Microphone array (for detecting a sound source and removing a noise)
(4) Bio-sensor signal sensor (for heart rate and oxygen saturation)

Further, emotional services applicable to the present disclosure are as follows.
(1) Artificial intelligence conversation that make patient's mind peaceful through a video call with sensibility anytime and anywhere
(2) Contact-free artificial intelligence emotional service by an agent (adoption of digital therapeutics such as stress decrease, early psychological counseling, mental health, recognition enhancement training, etc.)

Further, uses and applicable fields of services according ot the present disclosure are as follows.
(1) Object: negative pressure room, intensive care unit, general ward and screening station and home
(2) Video call between medical staff and patient's family
(3) Contact-free patient's condition monitoring and a summons
(4) Patient monitoring and prediction

### Utilization of patient care system through artificial intelligence-based monitoring using a plurality of apparatuses

FIG. 7 shows one example for the use of a patient care system through artificial intelligence-based monitoring using a plurality of apparatuses, in accordance with the present disclosure.

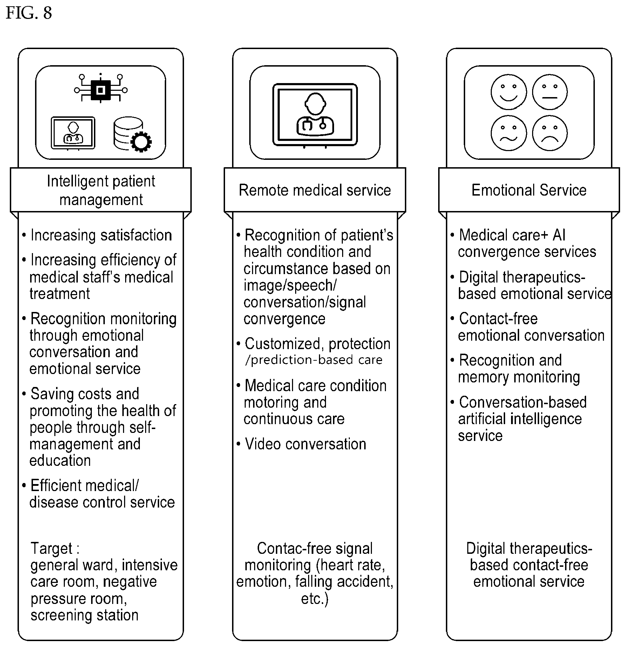

FIG. 8 shows use of service models according to the present disclosure.

According to FIGS. 7 and 8, obtaining of various kinds of data and uses thereof as follows are available through digital therapeutics-based emotional services.
- Emotion classification: obtaining data for a neutral emotion and six emotions (Happy, Sad, Surprise, Angry, Fear, Disgust according to Ekman)
- Regarding obtaining of the patient use data, since most of all, it is important to obtain a high quality of data in order to achieve a desired outcome using AI data, recognition/ emotional service qualities are improved by continuously obtaining patient use data mainly with a medical institute wherein a product is installed.
- Video call function: implemented by using a hand unit, and multiple microphones and cameras that are installed to a system
- Wide angle camera: increasing an accuracy of a facial expression

That is, the present disclosure has a progressive technical element through following elements.
(1) Medical loT-matched smart system and digital emotional service
(2) The technology of the present disclosure possesses a customized life healthcare service and a medical smart system capable of providing a remote medical service, while most of the home and foreign developments of remote medical services relates to a single service in a type of installed to many and unspecified smart phones.
   Particularly, this facilitates intensive and efficient medical management and approaching management due to the internal use by an institute, is available to obtain high quality materials and data, and allows efficient medical/disease control services.
(3) A new model of interactional typed intelligence patient management system may be provided, which is a "person-centered", particularly human emotion-based emotional service which existing medical apparatuses lack, allowing interactional systematic assistances between the human and artificial intelligence through "an artificial intelligent, natural conversation technology that is applied with human emotions and circumstances besides simple command-performance or question and answer".

### Patient care method through artificial intelligence-based monitoring

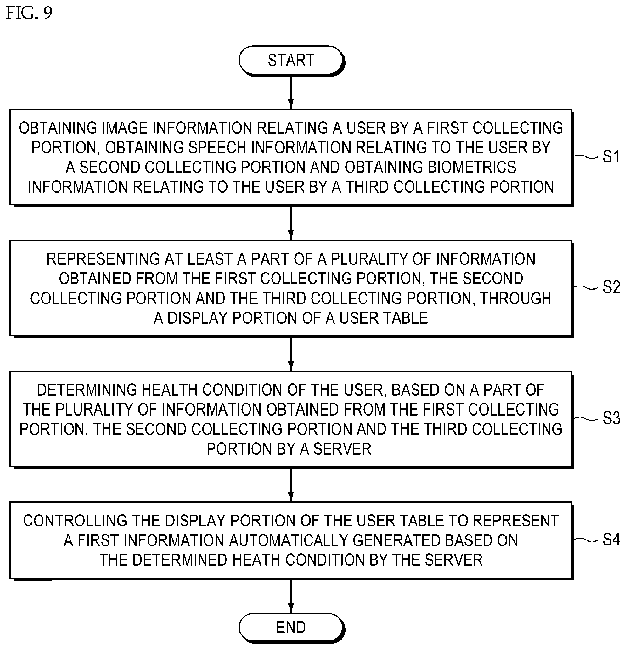

FIG. 9 shows a flowchart explaining a patient care method through artificial intelligence-based monitoring in accordance with the present disclosure.

Referring to FIG. 9, in step 1, image information relating a user is obtained by a first collecting portion, speech information relating to the user is obtained by a second collecting portion and biometrics information relating to the user is obtained by a third collecting portion.

Then, in step 2, at least a part of a plurality of information obtained from the first collecting portion, the second collecting portion and the third collecting portion is represented through a display portion 151 of a user table.

Further, in step 3, a server 200 determines health condition of the user based on a part of the plurality of information obtained from the first collecting portion, the second collecting portion and the third collecting portion.

Then, in step 4, the server 200 controls the display portion of the user table to represent a first information automatically generated based on the determined heath condition.

Meanwhile, the first information is converted in real time based on user's feedback on the first information and may represent a change in the determined health condition on the display portion.

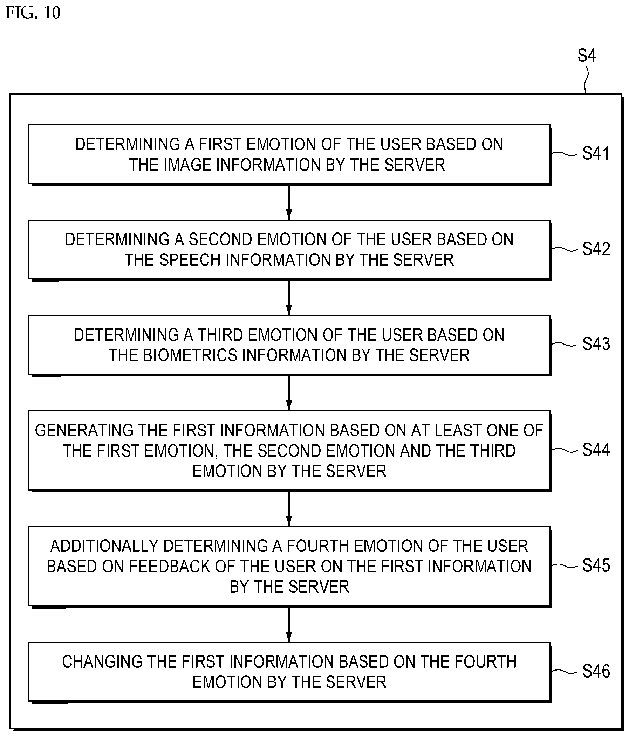

Meanwhile, FIG. 10 shows a flowchart of a method of monitoring based on emotion determination, in accordance with the present disclosure.

Referring to FIG. 10, in the step 4, firstly performed is step 41 in which the server determines a first emotion of the user based on the image information.

Then, in step 42, the server 200 determines a second emotion of the user based on the speech information.

Further, in step 43, the server 200 determines a third emotion of the user based on the biometrics information.

Then, in step 44, the server 200 may generate the first information based on at least one of the first emotion, the second emotion and the third emotion.

Further, in step 45, the server 200 may additionally determine a fourth emotion of the user based on feedback of the user on the first information.

Further, in step 46, the server 200 changes the first information based on the fourth emotion.

The server 200 determines the first emotion based on facial expression of the user, the second emotion based on speech melody of the user, and the fourth emotion based on contextual information on the feedback of the user.

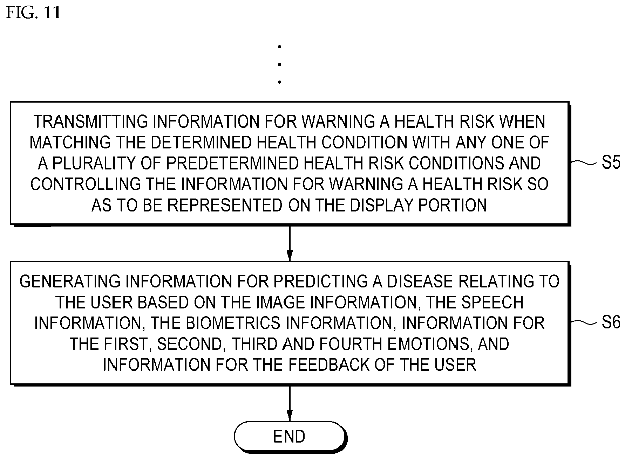

Meanwhile, FIG. 11 show a flowchart of a method for recognition of a risk situation and predicting user's disease, in accordance with the present disclosure.

Following the step 4 as described in FIG. 10, step 5 may be performed in which the server 200 transmits information for warning a health risk when matching the determined health condition with any one of a plurality of predetermined health risk conditions, and then controls the information for warning a health risk so as to be represented on the display portion.

Furthermore, following the step 5, step 6 may be performed in which the server 200 generates information for predicting a disease relating to the user based on the image information, the speech information, the biometrics information, information for the first, second, third and fourth emotions, and information for the feedback of the user.

### System using a band sensor

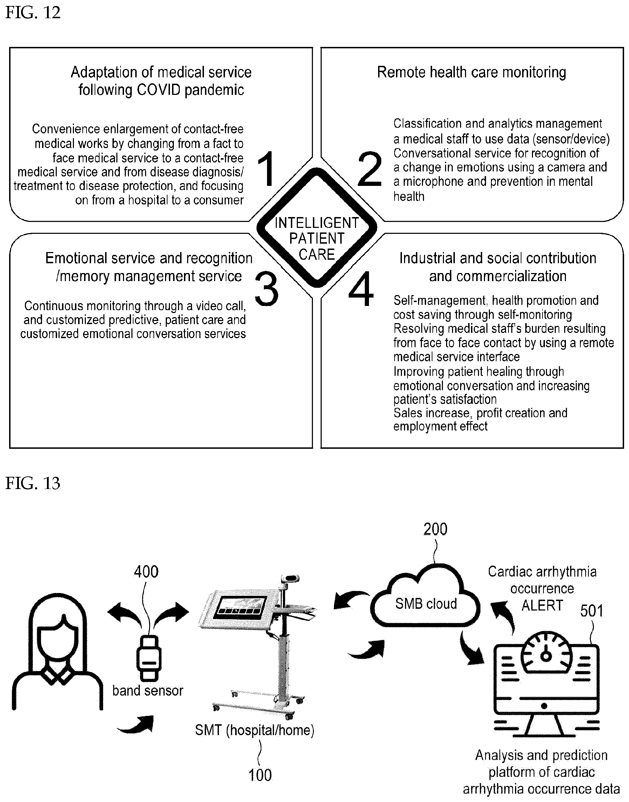

FIG. 13 shows another example for monitoring bio-signals using a band sensor.

Referring to FIG. 13, data for bio-signals may be continuously collected through a band sensor 400 and a user table 100 in a hospital and at home.

The data may be transmitted to and stored in the server 200 or a health cloud (not illustrated) through an SMT 100, and then analyzed and provided.

That is, information for a heart rate, heart rate variability (R-R interval), a breathing rate, activity and sleep may be analyzed through the server 200 and a predictive data analytics system 501.

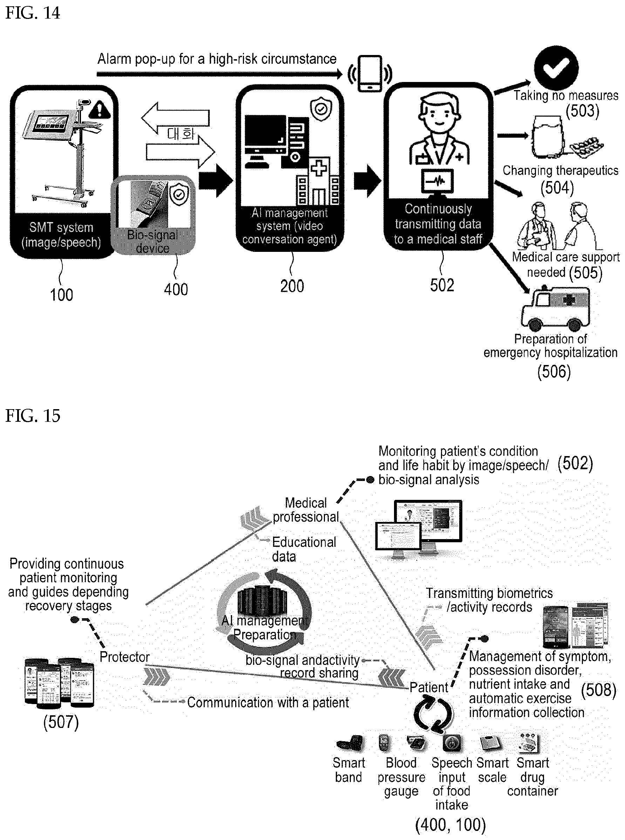

Further, FIG. 14 show a view explaining a scenario which a medical staff can take based on monitoring.

Referring to FIG. 14, an AI management system 200 analyzes data that is continuously extracted by a camera microphone and bio-signal monitoring 100, 400, performs conversation between a patient and a video conversational agent and transmits the analyzed content to a medical staff 502.

It, hereby, is capable of providing supports 503 to 506, for example, to a proper measure, a therapeutics change, a medical service support, a contingency plan, etc., reducing risk factors, and practicing advanced prevention by alarming for a high-risk circumstance through continuous monitoring.

As taking a side view of the patient, it is capable of reducing a death likely prevented, undergoing cost effective therapeutics, increasing compliance and performing easy monitoring.

Further, as considering an economical aspect, it is capable of early detecting HF decompensation failure and reducing the risk therefor, reducing hospitalization and re-hospitalization rates and providing efficient therapeutics.

Further as taking a side view of the medical staff, it is capable of performing a job with less workload and less real-time consultancy, rapidly discerning a required disease, setting up a potential medical parameter and understanding amelioration/aggravation trends of the disease condition through continuous monitoring.

FIG. 15 shows one embodiment according to the present disclosure.

Referring to FIG. 15, major events and health indexes are sorted out and then may be distributed to the medical staff 502 and patient's family periodically (for example: every week).

Further, it is available to transmit data among a patient 400, 100, a patient protector 507 and the medical staff 502 and to operate a management service platform 508.

Further, it is available to analyze correlation between ordinary events (for example, when carrying on ordinary conversation, etc.) and health indexes (HR, BR, stress, etc.), and causality relation therebetween.

Further, it is available to develop a predictive model through data analytics and to match a speech control technology.

### Advantageous effects according to the present disclosure

FIG. 12 shows a specific example for industrial effects according to the present disclosure.

Referring to FIG. 12, in order to solve the aforementioned existing problems, the present disclosure is capable of patient care methods and systems through artificial intelligence-based monitoring.

Particularly, the present disclosure is capable of providing patient care methods and systems through artificial intelligence-based monitoring which include a first collecting portion for obtaining image information relating a user, a second collecting portion for obtaining speech information relating to the user, a third collecting portion for obtaining biometrics information relating to the user, a user table having a display portion, and a server that determines health condition of the user, based on a part of the plurality of information obtained from the first collecting portion, the second collecting portion and the third collecting portion.

Particularly, according to the present disclosure, it is capable of representing a first information automatically generated based on determined health condition on the display portion of the user table, and of representing the first information changed in real time based on user's feedback and the determined health condition on the display portion.

According to the present disclosure, it is capable of providing an artificial intelligence emotional service which makes a patient restful to the spirit by applying image information(facial expression, etc.), speech information(speech melody, etc.), context(conversation context through speech recognition, etc.) and bio-signals(electrocardiogram, oxygen saturation, ect).

Further, according to the present disclosure, compared to existing commercialized speech recognition services, it is capable of saving medical expenses due to emotional stability resulting therefrom as adopting a conversational agent technology provided with human-like emotion and intelligent understanding of the situation (surrounding circumstances, mind, emotion, tendency, experience, intend, etc.) to medical services.

According to the present invention, it is capable of providing a system that communes with and monitors a patient and support their living in societies where shown are increases in aged persons and people who need emotional stability and where regions with the medical vacuum still exist.

According to the present invention, emotional services make contact-free medical treatment possible in the modern society where infectious diseases may spread, thereby allowing adopting remote medical treatment and AI medical assistants, so as to reduce medical staff's workload and infection risk.

According to the present disclosure, it is capable of accumulating data for individual's usual health/emotional states to provide the data to medical institutions, if necessary and of preliminary verifying the data through emotional services using questionnaires similar to those likely to be questioned by the medical staff when contact-free medical treatment and remote medical services are necessary, thereby reducing medical staff's workload.

According to the present disclosure, it is capable of increasing degree of completion of a conversational agent technology have to 'human-like emotion' and 'intelligent understanding of the situation' which satisfy patients, thereby being adopted to medical services.

Artificial intelligence requires for a technology that understands surrounding circumstances and user's mind (emotion, tendency, experience, intend, etc.) to help human beings. However, person's mind is delicately complex and hardly expressed outwardly. Thus, there are limits in increasing accuracy with noninvasive sensors (e.g. a camera, a microphone, an accelerometer, etc.). According to the present disclosure, it is capable of providing multi-modal learning data and a recognition technology, thereby distinguishing delicate differences.

Currently, in the country, healthcare monitoring is a new wave that is cannot avoidable and thus the study for establishment of a system is ongoing so as to secure competitiveness in the global market through deregulation according to external environment changing so rapidly. In addition, in foreign countries, existing face to face treatments cause the problem of costs. Furthermore, in advanced countries such as the Americas, Australia, etc., this causes big social issues and financial problems. Thus, those countries are in the progress of adopting telehealth to increasing efficiency and reduce medical expenses. Accordingly, it is expected that the present disclosure will have market power.

Expected effects according to the present disclosure are as follows.

### (1) Aspects of Science/Technology

- Personalized predictive and patient care technology through image/speech/bio-signal and artificial intelligence-based monitoring
- Securing a personalized, natural recognition monitoring technology through emotional conversation
- Enhancing IOT technology and improving versatility thereof
- Technology for encoding a speech and an image and transmitting a service
- Technology for patient monitoring, and data processing and utilization
- Prediction and management of a service result
- Obtaining Big data: obtaining and utilizing big data depending on wards, ages and disease
- Understanding characteristics depending on disease
- Obtaining characteristics of ethnic groups depending on respective countries
- Securing technology and design patents

### (2) Aspects of industry/society

- Resolving medical staff's burden resulting from face to face contact by using a conversation-based remote medical service interface
- Saving costs and promoting the health of people through patient's self-management and education
- Increasing personal life quality and reducing the social burden of disease
- Positive economic effects of social services and medical services
- Saving medical costs due to emotional stabilization
- Obtaining and utilizing big data

Meanwhile, advantageous effects to be obtained by the present disclosure are not limited to the aforementioned effects, and other not-mentioned advantageous effects may be clearly understood by those skilled in the art to which the present disclosure pertains from the description below.

Further, according to one embodiment of the present disclosure, it is capable of implementing the method described above as a code readable by a processor in a medium where a program is recorded. An examples of the medium include a ROM, a RAM, a CD-ROM, a magnetic tale, a floppy disc, an optical data storage device, etc., and further include one implemented into a type of carrier wave (for example, transmission via the internet).

The method and system as described above are not limited to the aforementioned embodiments and the whole or parts of the respective embodiments may be selectively combined, allowing various modifications for those embodiments to be made.

## Claims

1. A patient care system through artificial intelligence-based monitoring comprising:
a first collecting portion that obtains image information relating a user;
a second collecting portion that obtains speech information relating to the user;
a third collecting portion that obtains biometrics information relating to the user;
a user table has a display portion which represents at least a part of a plurality of information obtained from the first collecting portion, the second collecting portion and the third collecting portion; and
a server that determines health condition of the user, based on a part of the plurality of information obtained from the first collecting portion, the second collecting portion and the third collecting portion, wherein
the server controls the display portion of the user table to represent a first information automatically generated based on the determined heath condition,
the first information is changed in real time based on user's feedback on the first information and a change in the determined health condition, so as to be represented on the display portion.

2. The patient care system through artificial intelligence-based monitoring of claim 1, wherein
the server determines:
a first emotion of the user based on the image information;
a second emotion of the user based on the speech information; and
a third emotion of the user based on the biometrics information,
and generates the first information based on at least one of the first emotion, the second emotion and the third emotion.

3. The patient care system through artificial intelligence-based monitoring of claim 2, wherein
the server additionally determines a fourth emotion of the user based on feedback of the user on the first information,
and changes the first information based on the fourth emotion.

4. The patient care system through artificial intelligence-based monitoring of claim 3, wherein
the server determines:
the first emotion based on facial expression of the user;
the second emotion based on speech melody of the user; and
the fourth emotion based on contextual information on the feedback of the user.

5. The patient care system through artificial intelligence-based monitoring of claim 4, wherein
the server transmits information for warning a health risk when matching the determined health condition with any one of a plurality of predetermined health risk conditions, and controls the information for warning a health risk so as to be represented on the display portion.

6. The patient care system through artificial intelligence-based monitoring of claim 4, wherein
the server generates information for predicting a disease relating to the user based on the image information, the speech information, the biometrics information, information for the first, second, third and fourth emotions, and information for the feedback of the user.

7. The patient care system through artificial intelligence-based monitoring of claim 6, wherein
the server accumulates and stores the image information, the speech information, the biometrics information, the information for the first, second, third and fourth emotions, the information for the feedback of the user and the information for predicting a disease relating to the user,
and provides a plurality of the accumulated information to a predetermined medical institution.

8. The patient care system through artificial intelligence-based monitoring of claim 7, wherein
the patient care system through artificial intelligence-based monitoring is used in at least one of contact-free treatment and remote medical service.

9. The patient care system through artificial intelligence-based monitoring of claim 1, wherein
the first collecting portion comprises a camera, a wide angle camera and an infrared camera to take a photograph of the facial expression of the user, and
the image information is an image relating to the user at a negative pressure room, an intensive care unit, a general ward and a screening station and home.

10. The patient care system through artificial intelligence-based monitoring of claim 1,
the third collecting portion comprises: a smart watch that is worn on user's wrist and collects biometrics information relating to the user; an auditory information collecting portion that collects auditory information of the user; a gait information collecting portion that collects gait information of the user; a stress collecting portion that collects stress information of the user; an electrocardiogram(ECG) information collecting portion that collects ECG information of the user; a sleep information collection portion that collects sleep information of the user; a concentration information collecting portion that collects concentration information of the user; an electroencephalogram(EEG) information collection portion that collects EEG information of the user; an oxygen saturation information collecting portion that collects oxygen saturation formation of the user; and a temperature collecting portion that collects temperature information of the user.

11. The patient care system through artificial intelligence-based monitoring of claim 1, wherein
the user table comprises:
a panel portion that has a display pad;
a supporting frame that supports the panel portion; and
an angle-adjustable connecting portion that connects the panel portion and the supporting frame so as to adjust an angle of the panel portion at the supporting frame with an external force above a set force.

12. The patient care system through artificial intelligence-based monitoring of claim 11, wherein
the angle-adjustable connecting portion comprises:
a guide shaft portion that is fitted into the supporting frame;
a rotation bracket that is provided to the panel portion so as to fit the guide shaft portion thereinto; and
a binding guide portion that guides the rotation bracket so as to be bound movably along a circumference surface of the guide shaft portion.

13. A patient care method through artificial intelligence-based monitoring comprising steps of
Step 1 obtaining image information relating a user by a first collecting portion, obtaining speech information relating to the user by a second collecting portion and obtaining biometrics information relating to the user by a third collecting portion;
Step 2 representing at least a part of a plurality of information obtained from the first collecting portion, the second collecting portion and the third collecting portion, through a display portion of a user table;
Step 3 determining health condition of the user, based on a part of the plurality of information obtained from the first collecting portion, the second collecting portion and the third collecting portion by a server;
Step 4 controlling the display portion of the user table to represent a first information automatically generated based on the determined heath condition by the server, wherein
the first information is converted in real time based on user's feedback on the first information and represents a change in the determined health condition on the display portion.

14. The patient care system through artificial intelligence-based monitoring of claim 13, wherein
the step 4 comprises steps of:
Step 41 determining a first emotion of the user based on the image information by the server;
Step 42 determining a second emotion of the user based on the speech information by the server;
Step 43 determining a third emotion of the user based on the biometrics information by the server; and
Step 44 generating the first information based on at least one of the first emotion, the second emotion and the third emotion by the server; and
Step 45 additionally determining a fourth emotion of the user based on feedback of the user on the first information by the server; and
Step 46 changing the first information based on the fourth emotion by the server.

15. The patient care system through artificial intelligence-based monitoring of claim 14, wherein
the server determines the first emotion based on facial expression of the user, the second emotion based on speech melody of the user, and the fourth emotion based on contextual information on the feedback of the user, and
following the step 4, the server further comprises a step of:
Step 5 transmitting information for warning a health risk when matching the determined health condition with any one of a plurality of predetermined health risk conditions and controlling the information for warning a health risk so as to be represented on the display portion.

16. The patient care system through artificial intelligence-based monitoring of claim 15, wherein
following the step 5, the server further comprises a step of Step 6 generating information for predicting a disease relating to the user based on the image information, the speech information, the biometrics information, information for the first, second, third and fourth emotions, and information for the feedback of the user.
